# EUROPEAN PATENT APPLICATION

(11) **EP 4 265 589 A1**
(43) Date of publication of application: **25.10.2023**
(21) Application number: 21906736.0
(22) Date of filing: 17.12.2021
(51) Int. Cl.: C07C 17/02, C07C 19/10, C07C 22/08, C07C 67/307, C07C 69/63, C07C 69/635, C07C 69/76

(54) **METHOD FOR PRODUCING FLUORINATED ORGANIC COMPOUND**

(30) Priority: 18.12.2020 JP 2020210289
(71) Applicant: DAIKIN INDUSTRIES, LTD., Osaka-shi, Osaka 530-0001 (JP); SAGA UNIVERSITY, Saga-shi, Saga 840-8502 (JP)
(72) Inventor: HIGASHI, Masahiro, Osaka-shi, Osaka 530-0001 (JP); KISHIKAWA, Yosuke, Osaka-shi, Osaka 530-0001 (JP); KITAMURA, Tsugio, Osaka-shi, Osaka 530-0001 (JP); OYAMADA, Juzo, Osaka-shi, Osaka 530-0001 (JP)
(74) Representative: Hoffmann Eitle
(86) International application number: PCT/JP2021/046869
(87) International publication number: WO 2022/131378

(57) **Abstract**

The invention provides a novel method for producing a fluorinated organic compound. The method for producing a fluorinated organic compound comprises reacting a compound represented by formula (1): 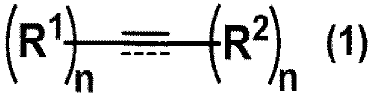 (wherein R¹ and R² are each independently a hydrogen atom, a halogen atom, or an organic group, or R¹ and R² optionally form a ring together with the two adjacent carbon atoms; n is 1 or 2;
and wherein two R¹s are optionally the same or different, two R²s are optionally the same or different, or two R¹s or two R²s optionally form a ring together with their adjacent carbon atom), with (A) at least one fluorine source selected from the group consisting of hydrogen fluoride, hydrogen fluoride salts, and fluoride salts, and (B) a halogen source other than fluorine represented by the formula: R³(OX)ₘ (wherein R³ is a hydrogen atom, a cation, or an organic group, X is a halogen atom other than a fluorine atom, and m is an integer corresponding to the valence of R³), to add fluorine and a halogen other than fluorine to the double bond or triple bond.

## Description

### Technical Field

The present disclosure relates to a method for producing a fluorinated organic compound.

### Background Art

Fluorinated organic compounds are extremely important compounds as various chemical products, such as functional materials, pharmaceutical and agrochemical compounds, and electronic materials, and as intermediates thereof.

For example, Non-patent Literature (NPL) 1 discloses a method for producing a fluorinated organic compound by reacting a hypohalite and BF₃ with an alkene in a nonpolar solvent of CH₂Cl₂ or CCl₄. NPL 2 discloses a method of reacting an alkene with hydrogen fluoride pyridine and N-halosuccinimide.

### Citation List

### Non-patent Literature

NPL 1: Heasley et al., Journal of Organic Chemistry, Vol. 48, No. 19, 1983, pp. 3195-3199
NPL 2: Olah et al., Journal of Organic Chemistry, Vol. 44, No. 22, 1979, pp. 3872-3881

### Summary of Invention

### Technical Problem

Since BF₃ in NPL 1 and N-halosuccinimide in NPL 2 are expensive, there is room for improvement in terms of production costs.

The problem to be solved by the present disclosure is to provide a novel method for producing a fluorinated organic compound.

### Solution to Problem

The present disclosure encompasses the following embodiments.

### Item 1.

A method for producing a halogen adduct of a compound represented by formula (1): 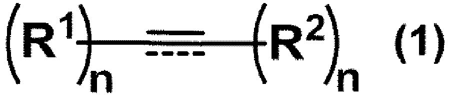 wherein
R¹ and R² are each independently a hydrogen atom, a halogen atom, or an organic group, or R¹ and R² optionally form a ring together with the two adjacent carbon atoms;
n is 1 or 2; and
the symbol 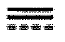 is a double bond or a triple bond,
with the proviso that
when the symbol is a triple bond, n is 1,
when the symbol is a double bond, n is 2,
two R¹s are optionally the same or different,
two R²s are optionally the same or different, or
two R¹s or two R²s optionally form a ring together with their adjacent carbon atom,
   the method comprising reacting the compound represented by formula (1) with

(A) at least one fluorine source selected from the group consisting of hydrogen fluoride, hydrogen fluoride salts, and fluoride salts, and
(B) a halogen source other than fluorine represented by the formula: R³(OX)ₘ, wherein R³ is a hydrogen atom, a cation, or an organic group, X is a halogen atom other than a fluorine atom, and m is an integer corresponding to the valence of R³, to add fluorine and a halogen other than fluorine to the double bond or triple bond.

### Item 2.

The production method according to Item 1, wherein the fluorine source (A) is at least one member selected from the group consisting of hydrogen fluoride, hydrogen fluoride amine salts, and alkali metal fluoride salts.

### Item 3.

The production method according to Item 1 or 2, wherein the amount of the fluorine source (A) is within the range of 0.1 to 1000 moles, per mole of the compound represented by formula (1) .

### Item 4.

The production method according to any one of Items 1 to 3, wherein in the halogen source (B) other than fluorine, R³ is an alkali metal, an alkaline earth metal, an alkyl group optionally having one or more substituents, a cycloalkyl group optionally having one or more substituents, an aryl group optionally having one or more substituents, a non-aromatic heterocyclic group optionally having one or more substituents, or a heteroaryl group optionally having one or more substituents. Item 5.

The production method according to any one of Items 1 to 4, wherein in the halogen source (B) other than fluorine, X is a chlorine atom.

### Item 6.

The production method according to any one of Items 1 to 5, wherein the halogen source (B) other than fluorine is represented by the formula: R^{3a}ClO, wherein R^{3a} is an alkali metal or a C₁-C₄ alkyl group.

### Item 7.

The production method according to any one of Items 1 to 6, wherein the amount of the halogen source (B) other than fluorine is within the range of 0.1 to 10 moles, per mole of the compound represented by formula (1).

### Item 8.

The production method according to any one of Items 1 to 7, wherein R¹ and R² are each independently a hydrogen atom, a halogen atom, an alkyl group optionally having one or more substituents, an alkoxy group optionally having one or more substituents, an alkylcarbonyl group optionally having one or more substituents, an alkylcarbonyloxy group optionally having one or more substituents, an alkoxycarbonyl group optionally having one or more substituents, a cycloalkyl group optionally having one or more substituents, an aryl group optionally having one or more substituents, or an aralkyl group optionally having one or more substituents, or R¹ and R² optionally form a ring together with the two adjacent carbon atoms.

### Item 9.

The production method according to Item 8, wherein the one or more substituents are at least one member selected from the group consisting of halo groups, a hydroxyl group, alkoxy groups, alkylcarbonyl groups, alkylcarbonyloxy groups, alkoxycarbonyl groups, arylcarbonyloxy groups, aryloxycarbonyl groups, and aryl groups.

### Item 10.

The production method according to any one of Items 1 to 9, wherein the compound represented by formula (1) is any one of the compounds represented by formulas (1a) to (1c): 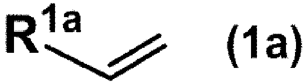 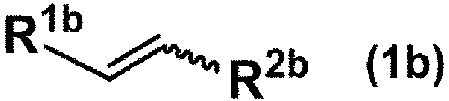 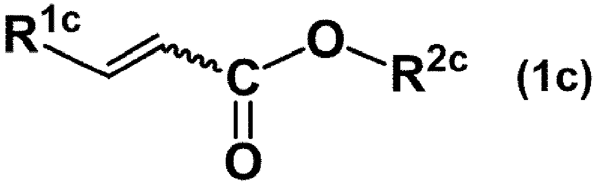 wherein
R^{1a} is an alkyl group optionally having one or more substituents, an aryl group optionally having one or more substituents, or an aralkyl group optionally having one or more substituents,
R^{1b} and R^{2b} are each independently an alkyl group optionally having one or more substituents, an aryl group optionally having one or more substituents, or an aralkyl group optionally having one or more substituents,
R^{1c} is a hydrogen atom, an alkyl group optionally having one or more substituents, an aryl group optionally having one or more substituents, or an aralkyl group optionally having one or more substituents, and
R^{2c} is an alkyl group optionally having one or more substituents. Item 11.

The production method according to any one of Items 1 to 10, wherein the reaction is performed in the presence of a solvent.

### Item 12.

The production method according to Item 11, wherein the solvent is at least one member selected from the group consisting of aliphatic hydrocarbons, aromatic hydrocarbons, halogenated hydrocarbons, ethers, esters, nitriles, and water.

### Item 13.

A halogenating agent comprising (A) and (B) below:
(A) at least one fluorine source selected from the group consisting of hydrogen fluoride, hydrogen fluoride salts, and fluoride salts; and
(B) a halogen source other than fluorine represented by the formula: R³(OX)ₘ, wherein R³ is a cation or an organic group, X is a halogen atom other than a fluorine atom, and m is an integer corresponding to the valence of R³.

### Advantageous Effects of Invention

The present disclosure provides a novel method for producing a fluorinated organic compound.

### Description of Embodiments

The above overview of the present disclosure is not intended to describe each of the disclosed embodiments or all of the implementations of the present disclosure.

The following description of the present disclosure more specifically exemplifies the embodiments of the examples.

In several parts of the present disclosure, guidance is provided through examples, and these examples can be used in various combinations.

In each case, the group of examples can function as a non-exclusive and representative group.

All publications, patents, and patent applications cited herein are incorporated herein by reference in their entirety.

### Terms

Unless otherwise specified, the symbols and abbreviations in the present specification can be understood in the context of the present specification in the meanings commonly used in the technical field to which the present disclosure belongs.

In the present specification, the terms "comprise" and "contain" are used with the intention of including the terms "consisting essentially of" and "consisting of."

Unless otherwise specified, the steps, treatments, or operations described in the present specification can be performed at room temperature.

In the present specification, room temperature can refer to a temperature within the range of 10 to 40°C.

In the present specification, the phrase "Cₙ₋ₘ" (n and m are each an integer of 1 or more) indicates that the number of carbon atoms is n or more and m or less, as can be generally understood by a person skilled in the art.

In the present specification, the phrase "compound represented by formula (N)" (N is an integer of 1 or more) can be referred to as "compound (N)."

In the present specification, unless otherwise specified, examples of the halogen atom may include fluorine, chlorine, bromine, and iodine.

In the present specification, the "organic group" refers to a group containing one or more carbon atoms.

Examples of the organic group may include
an alkyl group optionally having one or more substituents,
an alkenyl group optionally having one or more substituents,
an alkynyl group optionally having one or more substituents,
a cycloalkyl group optionally having one or more substituents,
a cycloalkenyl group optionally having one or more substituents,
a cycloalkadienyl group optionally having one or more substituents,
an aryl group optionally having one or more substituents,
an aralkyl group optionally having one or more substituents,
a non-aromatic heterocyclic group optionally having one or more substituents,
a heteroaryl group optionally having one or more substituents,
a cyano group,
an aldehyde group,
a carboxyl group,

   R^{r}O-,

   R^{r}CO- ,

   R^{r}COO- ,

   R^{r}SO₂- ,

   R^{r}OCO-,

   and

   R^{r}OSO₂-

   (in these formulas, R^{r} is independently
an alkyl group optionally having one or more substituents,
an alkenyl group optionally having one or more substituents,
an alkynyl group optionally having one or more substituents,
a cycloalkyl group optionally having one or more substituents,
a cycloalkenyl group optionally having one or more substituents,
a cycloalkadienyl group optionally having one or more substituents,
an aryl group optionally having one or more substituents,
an aralkyl group optionally having one or more substituents,
a non-aromatic heterocyclic group optionally having one or more substituents, or
a heteroaryl group optionally having one or more substituents).

In the present specification, unless otherwise specified, examples of the hydrocarbon group may include an alkyl group, an alkenyl group, an alkynyl group, a cycloalkyl group, a cycloalkenyl group, a cycloalkadienyl group, an aryl group, an aralkyl group, and a group that is a combination of these groups.

In the present specification, unless otherwise specified, examples of the alkyl group may include linear or branched C₁₋C₁₆ alkyl groups (e.g., C₁-C₁₄ alkyl groups and C₁-C₁₂ alkyl groups), such as methyl, ethyl, propyl, isopropyl, butyl, isobutyl, sec-butyl, tert-butyl, pentyl, isopentyl, neopentyl, hexyl, heptyl, octyl, nonyl, and decyl.

In the present specification, unless otherwise specified, examples of the alkenyl group may include linear or branched C₂-C₁₀ alkenyl groups, such as vinyl, 1-propen-1-yl, 2-propen-1-yl, isopropenyl, 2-buten-1-yl, 4-penten-1-yl, and 5-hexen-1-yl.

In the present specification, unless otherwise specified, examples of the alkynyl group may include linear or branched C₂-C₁₀ alkynyl groups, such as ethynyl, 1-propyn-1-yl, 2-propyn-1-yl, 4-pentyn-1-yl, and 5-hexyn-1-yl.

In the present specification, unless otherwise specified, examples of the cycloalkyl group may include C₃-C₇ cycloalkyl groups, such as cyclopropyl, cyclobutyl, cyclopentyl, cyclohexyl, and cycloheptyl.

In the present specification, unless otherwise specified, examples of the cycloalkenyl group may include C₃-C₇ cycloalkenyl groups, such as cyclopropenyl, cyclobutenyl, cyclopentenyl, cyclohexenyl, and cycloheptenyl.

In the present specification, unless otherwise specified, examples of the cycloalkadienyl group may include C₄-C₁₀ cycloalkadienyl groups, such as cyclobutadienyl, cyclopentadienyl, cyclohexadienyl, cycloheptadienyl, cyclooctadienyl, cyclononadienyl, and cyclodecadienyl.

In the present specification, unless otherwise specified, the aryl group may be monocyclic, bicyclic, tricyclic, or tetracyclic.

In the present specification, unless otherwise specified, the aryl group may be a C₆-C₁₈ aryl group.

In the present specification, unless otherwise specified, examples of the aryl group may include phenyl, 1-naphthyl, 2-naphthyl, 2-biphenyl, 3-biphenyl, 4-biphenyl, and 2-anthryl.

In the present specification, unless otherwise specified, examples of the aralkyl group may include benzyl, phenethyl, diphenylmethyl, 1-naphthylmethyl, 2-naphthylmethyl, 2,2-diphenylethyl, 3-phenylpropyl, 4-phenylbutyl, 5-phenylpentyl, 2-biphenylylmethyl, 3-biphenylylmethyl, and 4-biphenylylmethyl.

In the present specification, unless otherwise specified, the non-aromatic heterocyclic group may be monocyclic, bicyclic, tricyclic, or tetracyclic.

In the present specification, unless otherwise specified, the non-aromatic heterocyclic group may be, for example, a non-aromatic heterocyclic group containing, in addition to carbon, 1 to 4 heteroatoms selected from oxygen, sulfur, and nitrogen as a ring-constituting atom.

In the present specification, unless otherwise specified, the non-aromatic heterocyclic group may be saturated or unsaturated.

In the present specification, unless otherwise specified, examples of the non-aromatic heterocyclic group may include tetrahydrofuryl, oxazolidinyl, imidazolinyl (e.g., 1-imidazolinyl, 2-imidazolinyl, and 4-imidazolinyl), aziridinyl (e.g., 1-aziridinyl and 2-aziridinyl), azetidinyl (e.g., 1-azetidinyl and 2-azetidinyl), pyrrolidinyl (e.g., 1-pyrrolidinyl, 2-pyrrolidinyl, and 3-pyrrolidinyl), piperidinyl (e.g., 1-piperidinyl, 2-piperidinyl, and 3-piperidinyl), azepanyl (e.g., 1-azepanyl, 2-azepanyl, 3-azepanyl, and 4-azepanyl), azocanyl (e.g., 1-azocanyl, 2-azocanyl, 3-azocanyl, and 4-azocanyl), piperazinyl (e.g., 1,4-piperazin-1-yl and 1,4-piperazin-2-yl), diazepinyl (e.g., 1,4-diazepin-1-yl, 1,4-diazepin-2-yl, 1,4-diazepin-5-yl, and 1,4-diazepin-6-yl), diazocanyl (e.g., 1,4-diazocan-1-yl, 1,4-diazocan-2-yl, 1,4-diazocan-5-yl, 1,4-diazocan-6-yl, 1,5-diazocan-1-yl, 1,5-diazocan-2-yl, and 1,5-diazocan-3-yl), tetrahydropyranyl (e.g., tetrahydropyran-4-yl), morpholinyl (e.g., 4-morpholinyl), thiomorpholinyl (e.g., 4-thiomorpholinyl), 2-oxazolidinyl, dihydrofuryl, dihydropyranyl, dihydroquinolyl, and the like.

In the present specification, unless otherwise specified, examples of the heteroaryl group may include monocyclic aromatic heterocyclic groups (e.g., 5- or 6-membered monocyclic aromatic heterocyclic groups) and aromatic condensed heterocyclic groups (e.g., 5- to 18-membered aromatic condensed heterocyclic groups).

In the present specification, unless otherwise specified, examples of the 5- or 6-membered monocyclic aromatic heterocyclic groups may include pyrrolyl (e.g., 1-pyrrolyl, 2-pyrrolyl, and 3-pyrrolyl), furyl (e.g., 2-furyl and 3-furyl), thienyl (e.g., 2-thienyl and 3-thienyl), pyrazolyl (e.g., 1-pyrazolyl, 3-pyrazolyl, and 4-pyrazolyl), imidazolyl (e.g., 1-imidazolyl, 2-imidazolyl, and 4-imidazolyl), isoxazolyl (e.g., 3-isoxazolyl, 4-isoxazolyl, and 5-isoxazolyl), oxazolyl (e.g., 2-oxazolyl, 4-oxazolyl, and 5-oxazolyl), isothiazolyl (e.g., 3-isothiazolyl, 4-isothiazolyl, and 5-isothiazolyl), thiazolyl (e.g., 2-thiazolyl, 4-thiazolyl, and 5-thiazolyl), triazolyl (e.g., 1,2,3-triazol-4-yl and 1,2,4-triazol-3-yl), oxadiazolyl (e.g., 1,2,4-oxadiazol-3-yl and 1,2,4-oxadiazol-5-yl), thiadiazolyl (e.g., 1,2,4-thiadiazol-3-yl and 1,2,4-thiadiazol-5-yl), tetrazolyl, pyridyl (e.g., 2-pyridyl, 3-pyridyl, and 4-pyridyl), pyridazinyl (e.g., 3-pyridazinyl and 4-pyridazinyl), pyrimidinyl (e.g., 2-pyrimidinyl, 4-pyrimidinyl, and 5-pyrimidinyl), pyrazinyl, and the like.

In the present specification, unless otherwise specified, examples of the 5- to 18-membered aromatic condensed heterocyclic groups may include isoindolyl (e.g., 1-isoindolyl, 2-isoindolyl, 3-isoindolyl, 4-isoindolyl, 5-isoindolyl, 6-isoindolyl, and 7-isoindolyl), indolyl (e.g., 1-indolyl, 2-indolyl, 3-indolyl, 4-indolyl, 5-indolyl, 6-indolyl, and 7-indolyl), benzo[b]furanyl (e.g., 2-benzo[b]furanyl, 3-benzo[b]furanyl, 4-benzo[b]furanyl, 5-benzo[b]furanyl, 6-benzo[b]furanyl, and 7-benzo[b]furanyl), benzo[c]furanyl (e.g., 1-benzo[c]furanyl, 4-benzo[c]furanyl, and 5-benzo[c]furanyl), benzo[b]thienyl (e.g., 2-benzo[b]thienyl, 3-benzo[b]thienyl, 4-benzo[b]thienyl, 5-benzo[b]thienyl, 6-benzo[b]thienyl, and 7-benzo[b]thienyl), benzo[c]thienyl (e.g., 1-benzo[c]thienyl, 4-benzo[c]thienyl, and 5-benzo[c]thienyl), indazolyl (e.g., 1-indazolyl, 2-indazolyl, 3-indazolyl, 4-indazolyl, 5-indazolyl, 6-indazolyl, and 7-indazolyl), benzimidazolyl (e.g., 1-benzimidazolyl, 2-benzimidazolyl, 4-benzimidazolyl, and 5-benzimidazolyl), 1,2-benzisoxazolyl (e.g., 1,2-benzisoxazol-3-yl, 1,2-benzisoxazol-4-yl, 1,2-benzisoxazol-5-yl, 1,2-benzisoxazol-6-yl, and 1,2-benzisoxazol-7-yl), benzoxazolyl (e.g., 2-benzoxazolyl, 4-benzoxazolyl, 5-benzoxazolyl, 6-benzoxazolyl, and 7-benzoxazolyl), 1,2-benzisothiazolyl (e.g., 1,2-benzisothiazol-3-yl, 1,2-benzisothiazol-4-yl, 1,2-benzisothiazol-5-yl, 1,2-benzisothiazol-6-yl, and 1,2-benzisothiazol-7-yl), benzothiazolyl

(e.g., 2-benzothiazolyl, 4-benzothiazolyl, 5-benzothiazolyl, 6-benzothiazolyl, and 7-benzothiazolyl), isoquinolyl (e.g., 1-isoquinolyl, 3-isoquinolyl, 4-isoquinolyl, and 5-isoquinolyl), quinolyl (e.g., 2-quinolyl, 3-quinolyl, 4-quinolyl, 5-quinolyl, and 8-quinolyl), cinnolinyl (e.g., 3-cinnolinyl, 4-cinnolinyl, 5-cinnolinyl, 6-cinnolinyl, 7-cinnolinyl, and 8-cinnolinyl), phthalazinyl (e.g., 1-phthalazinyl, 4-phthalazinyl, 5-phthalazinyl, 6-phthalazinyl, 7-phthalazinyl, and 8-phthalazinyl), quinazolinyl (e.g., 2-quinazolinyl, 4-quinazolinyl, 5-quinazolinyl, 6-quinazolinyl, 7-quinazolinyl, and 8-quinazolinyl), quinoxalinyl (e.g., 2-quinoxalinyl, 3-quinoxalinyl, 5-quinoxalinyl, 6-quinoxalinyl, 7-quinoxalinyl, and 8-quinoxalinyl), pyrazolo[1,5-a]pyridyl (e.g., pyrazolo[1,5-a]pyridin-2-yl, pyrazolo[1,5-a]pyridin-3-yl, pyrazolo[1,5-a]pyridin-4-yl, pyrazolo[1,5-a]pyridin-5-yl, pyrazolo[1,5-a]pyridin-6-yl, and pyrazolo[1,5-a]pyridin-7-yl), imidazo[1,2-a]pyridyl (e.g., imidazo[1,2-a]pyridin-2-yl, imidazo[1,2-a]pyridin-3-yl, imidazo[1,2-a]pyridin-5-yl, imidazo[1,2-a]pyridin-6-yl, imidazo[1,2-a]pyridin-7-yl, and imidazo[1,2-a]pyridin-8-yl).

In the present specification, examples of R^{r}O- may include alkoxy (e.g., C₁-C₁₀ alkoxy, such as methoxy, ethoxy, propoxy, and butoxy), cycloalkoxy (e.g., C₃-C₇ cycloalkoxy, such as cyclopentoxy and cyclohexoxy), aryloxy (e.g., C₆-C₁₈ aryloxy, such as phenoxy and naphthoxy), and aralkyloxy (e.g., C₇-C₁₉ aralkyloxy, such as benzyloxy and phenethyloxy).

In the present specification, examples of R^{r}CO- may include alkylcarbonyl (e.g., (C₁-C₁₀ alkyl)carbonyl, such as acetyl, propionyl, and butyryl), cycloalkylcarbonyl (e.g., (C₃-C₇ cycloalkyl)carbonyl, such as cyclopentanoyl and cyclohexanoyl), arylcarbonyl (e.g., (C₆-C₁₈ aryl)carbonyl, such as benzoyl and naphthoyl), and aralkylcarbonyl (e.g., (C₇-C₁₉ aralkyl)carbonyl, such as benzylcarbonyl and phenethylcarbonyl).

In the present specification, examples of R^{r}COO- may include alkylcarbonyloxy (e.g., (C₁-C₁₀ alkyl)carbonyloxy, such as acetyloxy, propionyloxy, and butyryloxy), cycloalkylcarbonyloxy (e.g., (C₃-C₇ cycloalkyl)carbonyloxy, such as cyclopentanoyloxy and cyclohexanoyloxy), arylcarbonyloxy (e.g., (C₆-C₁₈ aryl)carbonyloxy, such as benzoyloxy and naphthoyloxy), and aralkylcarbonyloxy (e.g., (C₇-C₁₉ aralkyl)carbonyloxy, such as benzylcarbonyloxy and phenethylcarbonyloxy).

In the present specification, examples of R^{r}SO₂- may include alkylsulfonyl (e.g., C₁-C₁₀ alkylsulfonyl, such as methylsulfonyl, ethylsulfonyl, and propylsulfonyl), cycloalkylsulfonyl (e.g., C₄-C₈ cycloalkylsulfonyl, such as cyclopentylsulfonyl and cyclohexylsulfonyl), arylsulfonyl (e.g., C₆-C₁₈ arylsulfonyl, such as phenylsulfonyl and naphthylsulfonyl), and aralkylsulfonyl (e.g., C₇-C₁₉ aralkylsulfonyl, such as benzylsulfonyl and phenethylsulfonyl).

In the present specification, examples of R^{r}OCO- may include alkoxycarbonyl (e.g., C₁-C₁₀ alkoxy)carbonyl, such as methoxycarbonyl, ethoxycarbonyl, and propoxycarbonyl), cycloalkoxycarbonyl (e.g., (C₃-C₇ cycloalkoxy)carbonyl, such as cyclopentoxycarbonyl and cyclohexoxycarbonyl), aryloxycarbonyl (e.g., (C₆-C₁₈ aryloxy)carbonyl, such as phenoxycarbonyl and naphthoxycarbonyl), and aralkyloxycarbonyl (e.g., (C₇-C₁₉ aralkyloxy)carbonyl, such as benzyloxycarbonyl and phenethyloxycarbonyl.

In the present specification, examples of R^{r}OSO₂- may include alkoxysulfonyl (e.g., C₁-C₁₀ alkoxysulfonyl, such as methoxysulfonyl, ethoxysulfonyl, and propoxysulfonyl), cycloalkoxysulfonyl (e.g., C₃-C₇ cycloalkoxysulfonyl, such as cyclopentoxysulfonyl and cyclohexoxysulfonyl), aryloxysulfonyl (e.g., C₆-C₁₈ aryloxysulfonyl, such as phenoxysulfonyl and naphthoxysulfonyl), and aralkyloxysulfonyl (e.g., C₇-C₁₉ aralkyloxysulfonyl, such as benzyloxysulfonyl and phenethyloxysulfonyl) .

In the present specification, examples of the substituents in the "hydrocarbon group optionally having one or more substituents," "alkyl group optionally having one or more substituents," "alkenyl group optionally having one or more substituents," "alkynyl group optionally having one or more substituents," "cycloalkyl group optionally having one or more substituents," "cycloalkenyl group optionally having one or more substituents," "cycloalkadienyl group optionally having one or more substituents," "aryl group optionally having one or more substituents," "aralkyl group optionally having one or more substituents," "non-aromatic heterocyclic group optionally having one or more substituents," and "heteroaryl group optionally having one or more substituents" may include a halo group, a nitro group, a cyano group, an oxo group, a thioxo group, a carboxyl group, a sulfo group, a sulfamoyl group, a sulfinamoyl group, a sulfenamoyl group, R^{r}O-, R^{r}CO-, R^{r}COO-, R^{r}SO₂-, R^{r}OCO-, and R^{r}OSO₂- (in these formulas, R^{r} is as defined above) .

Of these substituents, examples of the halo group may include fluoro, chloro, bromo, and iodo.

The number of substituents may be within the range of 1 to the maximum substitutable number (e.g., 1, 2, 3, 4, 5, or 6).

### Method for Producing Halogen Adduct of Compound (1)

The production method according to the present disclosure comprises reacting compound (1) with fluorine source (A) and halogen source (B) other than fluorine to add fluorine and a halogen other than fluorine to the double bond or triple bond of compound (1).

### Reaction Substrate: Compound (1)

In one embodiment of compound (1),
preferably, R¹ and R² are each independently a hydrogen atom, a halogen atom, an alkyl group optionally having one or more substituents, an alkoxy group optionally having one or more substituents, an alkylcarbonyl group optionally having one or more substituents, an alkylcarbonyloxy group optionally having one or more substituents, an alkoxycarbonyl group optionally having one or more substituents, a cycloalkyl group optionally having one or more substituents, an aryl group optionally having one or more substituents, or an aralkyl group optionally having one or more substituents, and
more preferably, R¹ and R² are each independently a hydrogen atom, a halogen atom, an alkyl group optionally substituted with one or more halo groups, an alkoxy group optionally substituted with one or more halo groups, an alkoxyalkyl group optionally substituted with one or more halo groups, an alkylcarbonyl group optionally substituted with one or more halo groups, an alkylcarbonyloxy group optionally substituted with one or more halo groups, an alkoxycarbonyl group optionally substituted with one or more halo groups, a cycloalkyl group optionally having one or more substituents an aryl group optionally having one or more substituents, or an aralkyl group optionally having one or more substituents.

The substituents may be any group having a structure that enables substitution with the target and may be, for example, at least one member selected from the group consisting of halo groups, a hydroxyl group, alkoxy groups, alkylcarbonyl groups, alkylcarbonyloxy groups, alkoxycarbonyl groups, arylcarbonyloxy groups, aryloxycarbonyl groups, and aryl groups. The number of substituents may be within the range of 1 to the maximum substitutable number (e.g., 1, 2, or 3).

In one embodiment, R¹ and R² may form a ring together with the two adjacent carbon atoms. The ring is formed generally when the symbol is a double bond and n is 2.

Examples of the ring include cycloalkene rings corresponding to the groups mentioned above as examples of the "cycloalkenyl group," and non-aromatic heterocyclic rings corresponding to the groups with carbon-carbon double bond(s) among the groups mentioned above as examples of the "non-aromatic heterocyclic group."

The ring optionally has one or more substituents.

Examples of the substituents may include a hydrocarbon group, a halo group, a nitro group, a cyano group, an oxo group, a thioxo group, a carboxyl group, a sulfo group, a sulfamoyl group, a sulfinamoyl group, a sulfenamoyl group, R^{r}O-, R^{r}CO-, R^{r}COO-, R^{r}SO₂-, R^{r}OCO-, and R^{r}OSO₂- (in these formulas, R^{r} is as defined above).

The number of substituents may be within the range of 1 to the maximum substitutable number (e.g., 1, 2, 3, 4, or 5).

Preferable examples of compound (1) include a compound represented by formula (1A): 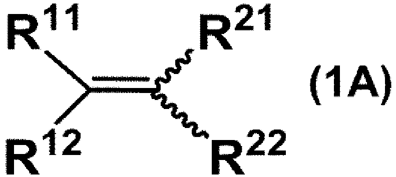 wherein
R¹¹, R¹², R²¹, and R²² are each independently a hydrogen atom, a halogen atom, or an organic group, R¹¹ and R¹², or R²¹ and R²² optionally form a ring together with the one adjacent carbon atom, or R¹¹ and R²¹, R¹¹ and R²², R¹² and R²¹, or R¹² and R²² optionally form a ring together with the two adjacent carbon atoms;
the symbol 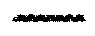 indicates a *cis* configuration or a *trans* configuration; and
a compound represented by formula (1B): 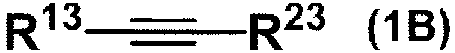 wherein R¹³ and R²³ are each independently a hydrogen atom, a halogen atom, or an organic group.

In one embodiment of compound (1A), preferably, R¹¹, R¹², and R²¹ are each independently a hydrogen atom, a halogen atom, an alkyl group optionally having one or more substituents, an alkoxy group optionally having one or more substituents, an alkylcarbonyl group optionally having one or more substituents, an alkylcarbonyloxy group optionally having one or more substituents, an alkoxycarbonyl group optionally having one or more substituents, a cycloalkyl group optionally having one or more substituents, an aryl group optionally having one or more substituents, or an aralkyl group optionally having one or more substituents, and R²² is a hydrogen atom, and more preferably, R¹¹, R¹², and R²¹ are each independently a hydrogen atom, a halogen atom, an alkyl group optionally substituted with one or more halo groups, an alkoxy group optionally substituted with one or more halo groups, an alkoxyalkyl group optionally substituted with one or more halo groups, an alkylcarbonyl group optionally substituted with one or more halo groups, an alkylcarbonyloxy group optionally substituted with one or more halo groups, an alkoxycarbonyl group optionally substituted with one or more halo groups, a cycloalkyl group optionally having one or more substituents an aryl group optionally having one or more substituents, or an aralkyl group optionally having one or more substituents, and R²² is a hydrogen atom.

In the above embodiment,
preferably, R¹¹ and R²¹ are each independently a hydrogen atom, a halogen atom, an alkyl group optionally having one or more substituents, an alkoxy group optionally having one or more substituents, an alkylcarbonyl group optionally having one or more substituents, an alkylcarbonyloxy group optionally having one or more substituents, an alkoxycarbonyl group optionally having one or more substituents, a cycloalkyl group optionally having one or more substituents, an aryl group optionally having one or more substituents, or an aralkyl group optionally having one or more substituents, and R¹² and R²² are each a hydrogen atom, and
more preferably, R¹¹ and R²¹ are each independently a hydrogen atom, a halogen atom, an alkyl group optionally substituted with one or more halo groups, an alkoxy group optionally substituted with one or more halo groups, an alkoxyalkyl group optionally substituted with one or more halo groups, an alkylcarbonyl group optionally substituted with one or more halo groups, an alkylcarbonyloxy group optionally substituted with one or more halo groups, an alkoxycarbonyl group optionally substituted with one or more halo groups, a cycloalkyl group optionally having one or more substituents an aryl group optionally having one or more substituents, or an aralkyl group optionally having one or more substituents, and R¹² and R²² are each a hydrogen atom.

In the above embodiment,
preferably R¹¹ is a hydrogen atom, a halogen atom, an alkyl group optionally having one or more substituents, an alkoxy group optionally having one or more substituents, an alkylcarbonyl group optionally having one or more substituents, an alkylcarbonyloxy group optionally having one or more substituents, an alkoxycarbonyl group optionally having one or more substituents, a cycloalkyl group optionally having one or more substituents, an aryl group optionally having one or more substituents, or an aralkyl group optionally having one or more substituents, R²¹ is a hydrogen atom, an alkyl group optionally having one or more substituents, a cycloalkyl group optionally having one or more substituents, an aryl group optionally having one or more substituents, or an aralkyl group optionally having one or more substituents, and R¹² and R²² are each a hydrogen atom, and
more preferably, R¹¹ is a hydrogen atom, a halogen atom, an alkyl group optionally substituted with one or more halo groups, an alkoxy group optionally substituted with one or more halo groups, an alkoxyalkyl group optionally substituted with one or more halo groups, an alkylcarbonyl group optionally substituted with one or more halo groups, an alkylcarbonyloxy group optionally substituted with one or more halo groups, an alkoxycarbonyl group optionally substituted with one or more halo groups, a cycloalkyl group optionally having one or more substituents an aryl group optionally having one or more substituents, or an aralkyl group optionally having one or more substituents, R²¹ is a hydrogen atom, an alkyl group optionally having one or more substituents, a cycloalkyl group optionally having one or more substituents, an aryl group optionally having one or more substituents, or an aralkyl group optionally having one or more substituents, and R¹² and R²² are each a hydrogen atom.

In the above embodiment, the substituents may be any group having a structure that enables substitution with the target and may be, for example, at least one member selected from the group consisting of halo groups, a hydroxyl group, alkoxy groups, alkylcarbonyl groups, alkylcarbonyloxy groups, alkoxycarbonyl groups, arylcarbonyloxy groups, aryloxycarbonyl groups, and aryl groups. The number of substituents may be within the range of 1 to the maximum substitutable number (e.g., 1, 2, or 3) .

In one embodiment of compound (1A), R¹¹ and R¹² or R²¹ and R²² optionally form a ring together with the one adjacent carbon atom. Examples of the ring include cycloalkane rings corresponding to the groups mentioned above as examples of the "cycloalkyl group" (e.g., a C₅-C₇ cycloalkane ring, such as a cyclohexane ring), and non-aromatic heterocyclic rings corresponding to the groups mentioned above as examples of the "non-aromatic heterocyclic group." The ring optionally has one or more substituents.

Examples of the substituents may include a hydrocarbon group, a halo group, a nitro group, a cyano group, an oxo group, a thioxo group, a carboxyl group, a sulfo group, a sulfamoyl group, a sulfinamoyl group, a sulfenamoyl group, R^{r}O-, R^{r}CO-, R^{r}COO-, R^{r}SO₂-, R^{r}OCO-, and R^{r}OSO₂- (in these formulas, R^{r} is as defined above).

The number of substituents may be within the range of 1 to the maximum substitutable number (e.g., 1, 2, 3, 4, or 5).

In one embodiment of compound (1A), R¹¹ and R²¹, R¹¹ and R²², R¹² and R²¹, or R¹² and R²² optionally form a ring together with the two adjacent carbon atoms. Examples of the ring include cycloalkene rings corresponding to the groups mentioned above as examples of the "cycloalkenyl group" (e.g., a C₅-C₇ cycloalkene ring, such as a cyclohexene ring), and non-aromatic heterocyclic rings corresponding to the groups with carbon-carbon double bond(s) among the groups mentioned above as examples of the "non-aromatic heterocyclic group."

The ring optionally has one or more substituents.

Examples of the substituents may include a hydrocarbon group, a halo group, a nitro group, a cyano group, an oxo group, a thioxo group, a carboxyl group, a sulfo group, a sulfamoyl group, a sulfinamoyl group, a sulfenamoyl group, R^{r}O-, R^{r}CO-, R^{r}COO-, R^{r}SO₂-, R^{r}OCO-, and R^{r}OSO₂- (in these formulas, R^{r} is as defined above).

The number of substituents may be within the range of 1 to the maximum substitutable number (e.g., 1, 2, 3, 4, or 5).

Preferable examples of compound (1A) include compounds represented by formulas (1a) to (1c): 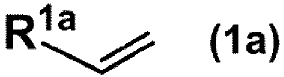 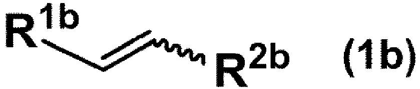 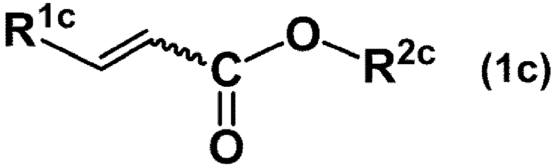 wherein
R^{1a} is an alkyl group optionally having one or more substituents, an aryl group optionally having one or more substituents, or an aralkyl group optionally having one or more substituents,
R^{1b} and R^{2b} are each independently an alkyl group optionally having one or more substituents, an aryl group optionally having one or more substituents, or an aralkyl group optionally having one or more substituents,
R^{1c} is a hydrogen atom, an alkyl group optionally having one or more substituents, an aryl group optionally having one or more substituents, or an aralkyl group optionally having one or more substituents, and
R^{2c} is an alkyl group optionally having one or more substituents.

The substituents may be any group having a structure that enables substitution with the target and may be, for example, at least one member selected from the group consisting of halo groups, a hydroxyl group, alkoxy groups, alkylcarbonyl groups, alkylcarbonyloxy groups, alkoxycarbonyl groups, arylcarbonyloxy groups, aryloxycarbonyl groups, and aryl groups. The number of substituents may be within the range of 1 to the maximum substitutable number (e.g., 1, 2, or 3).

In one embodiment of compound (1a), preferably, R^{1a} is an alkyl group optionally having one or more substituents or an aralkyl group optionally having one or more substituents.

In one embodiment of compound (1b), preferably, R^{1b} and R^{2b} are each independently an aryl group optionally having one or more substituents.

In one embodiment of compound (1c), preferably, R^{1c} is a hydrogen atom, an alkyl group optionally having one or more substituents, or an aryl group optionally having one or more substituents, and R^{2c} is an alkyl group optionally having one or more substituents.

In one embodiment of compound (1B),
preferably, R¹³ and R²³ are each independently a hydrogen atom, a halogen atom, an alkyl group optionally having one or more substituents, an alkoxy group optionally having one or more substituents, an alkylcarbonyl group optionally having one or more substituents, an alkylcarbonyloxy group optionally having one or more substituents, an alkoxycarbonyl group optionally having one or more substituents, a cycloalkyl group optionally having one or more substituents, an aryl group optionally having one or more substituents, or an aralkyl group optionally having one or more substituents, and
more preferably, R¹³ and R²³ are each independently a hydrogen atom, a halogen atom, an alkyl group optionally substituted with one or more halo groups, an alkoxy group optionally substituted with one or more halo groups, an alkoxyalkyl group optionally substituted with one or more halo groups, an alkylcarbonyl group optionally substituted with one or more halo groups, an alkylcarbonyloxy group optionally substituted with one or more halo groups, an alkoxycarbonyl group optionally substituted with one or more halo groups, a cycloalkyl group optionally having one or more substituents an aryl group optionally having one or more substituents, or an aralkyl group optionally having one or more substituents.

In the above embodiment, the substituents may be any group having a structure that enables substitution with the target and may be, for example, at least one member selected from the group consisting of halo groups, a hydroxyl group, alkoxy groups, alkylcarbonyl groups, alkylcarbonyloxy groups, alkoxycarbonyl groups, arylcarbonyloxy groups, aryloxycarbonyl groups, and aryl groups. The number of substituents may be within the range of 1 to the maximum substitutable number (e.g., 1, 2, or 3) .

### Fluorine Source (A)

Of fluorine sources (A), hydrogen fluoride may be used as an aqueous solution (hydrofluoric acid). The aqueous solution may be, for example, an aqueous solution having a hydrogen fluoride concentration of 10 to 70 mass%.

Of fluorine sources (A), examples of hydrogen fluoride salts include hydrogen fluoride amine salts and hydrogen fluoride ammonium salts.

In the hydrogen fluoride amine salts, the amine may be a chain amine or a cyclic amine.

Examples of chain amines include aliphatic primary amines, aliphatic secondary amines, and aliphatic tertiary amines.

Examples of aliphatic primary amines include C₁-C₆ alkylamines, such as methylamine, ethylamine, propylamine, butylamine, pentylamine, and hexylamine.

Examples of aliphatic secondary amines include di-C₁-C₆ alkylamines, such as dimethylamine, diethylamine, dipropylamine, dibutylamine, dipentylamine, and dihexylamine.

Examples of aliphatic tertiary amines include tri-C₁-C₆ alkylamines, such as trimethylamine, triethylamine, diisopropylethylamine, tributylamine, and N,N,N',N'-tetramethylethylenediamine.

Examples of cyclic amines include aliphatic cyclic amines and aromatic cyclic amines.

Examples of aliphatic cyclic amines include piperidine, piperazine, pyrrolidine, morpholine, N-methylpiperazine, N-methylpyrrolidine, 5-diazabicyclo[4.3.0]non-5-ene, and 1,4-diazabicyclo[2.2.2]octane.

Examples of aromatic cyclic amines include pyridine, pyrimidine, pyrazine, quinoline, and imidazole.

Examples of hydrogen fluoride ammonium salts include a compound represented by formula (A1):

NQ₄•xHF (A1)

wherein Q is each independently a hydrogen atom, an alkyl group, a cycloalkyl group, an aryl group, or an aralkyl group, and x is an integer of 1 or more.

In one embodiment of compound (A1), Q is preferably an alkyl group, and more preferably a C₁-C₆ alkyl group. X is preferably 1 to 10.

Preferable examples of compound (A1) include ammonium hydrogen fluoride (NH₄F•HF), hydrogen fluoride-tetramethylammonium fluoride, hydrogen fluoride-tetraethylammonium fluoride, hydrogen fluoride-tetrapropylammonium fluoride, and hydrogen fluoride-tetrabutylammonium fluoride.

Of fluoride sources (A), example of fluoride salts include a compound represented by formula (A2):

M¹Fₘ₁ (A2)

wherein M¹ is an alkali metal or an alkaline earth metal; and
m1 is 1 or 2, depending on the valence of M¹.

In one embodiment of compound (A2),
M¹ is preferably Li, Na, K, Ca, or Cs,
M¹ is more preferably Na, K, or Ca,
and M¹ is still more preferably K.

The fluoride salts may be preferably alkali metal fluoride salts (compounds (A2) in which M¹ is an alkali metal, and m1 is 1).

Fluorine sources (A) may be used singly or in a combination of two or more.

The amount of fluorine source (A) used is not limited. The lower limit of the amount of fluorine source (A) used can be, for example, 0.1 moles or more, preferably 0.2 moles or more, more preferably 0.3 moles or more, even more preferably 0.4 moles or more, and particularly preferably 0.5 moles or more, per mole of compound (1). The upper limit of the amount of fluorine source (A) used can be, for example, 1000 moles or less, preferably 500 moles or less, more preferably 300 moles or less, even more preferably 100 moles or less, still more preferably 50 moles or less, and particularly preferably 10 moles or less, per mole of compound (1). The amount of fluorine source (A) used can be, for example, within the range of 0.1 to 1000 moles, preferably within the range of 0.2 to 500 moles, more preferably within the range of 0.3 to 100 moles, even more preferably within the range of 0.4 to 50 moles, and particularly preferably within the range of 0.5 to 10 moles.

Fluorine source (A) remaining after the reaction may be trapped and discarded; however, it is preferable to recover and reuse it from the viewpoint of production costs. The trapped residue after the reaction may be washed with, for example, water or alkaline water.

### Halogen Source (B) Other Than Fluorine

In one embodiment of halogen source (B),
preferably, R³ is an alkali metal, an alkaline earth metal, an alkyl group optionally having one or more substituents, a cycloalkyl group optionally having one or more substituents, an aryl group optionally having one or more substituents, a non-aromatic heterocyclic group optionally having one or more substituents, or a heteroaryl group optionally having one or more substituents.

Examples of the alkali metals include Li, Na, K, and Cs. Examples of the alkaline earth metals include Mg and Ca.

The substituents may be any group having a structure that enables substitution with the target and may be, for example, a halo group. The number of substituents may be within the range of 1 to the maximum substitutable number (e.g., 1, 2, or 3).

R³ is more preferably an alkali metal or an alkyl group optionally having one or more substituents,
R³ is even more preferably an alkali metal or an alkyl group,
R³ is still more preferably an alkali metal or a C₁-C₆ alkyl group, and
R³ is particularly preferably an alkali metal or a C₁-C₄ alkyl group.

In one embodiment of halogen source (B), X is preferably a chlorine atom, a bromine atom, or an iodine atom, X is more preferably a chlorine atom or a bromine atom, and X is even more preferably a chlorine atom.

Halogen source (B) may be, for example, a hydrate.

Preferable examples of halogen source (B) include a compound represented by formula (B1):

R^{3a}ClO (B1)

wherein R^{3a} is an alkali metal or a C₁-C₄ alkyl group.

Halogen sources (B) may be used singly or in a combination of two or more.

The amount of halogen source (B) used is not limited. The lower limit of the amount of halogen source (B) used can be, for example, 0.1 moles or more, preferably 0.2 moles or more, and even more preferably 0.3 moles or more, per mole of compound (1). The lower limit of the amount of halogen source (B) used can be, for example, 10 moles or less, preferably 9 moles or less, and even more preferably 8 moles or less, per mole of compound (1). The amount of halogen source (B) used can be, for example, within the range of 0.1 to 10 moles, preferably 0.2 to 9 moles, and more preferably 0.3 to 8 moles, per mole of compound (1).

Halogen source (B) remaining after the reaction may be trapped and discarded; however, it is preferable to recover and reuse it from the viewpoint of production costs. The trapped residue after the reaction may be washed with, for example, water or alkaline water.

### BF₃ and N-halosuccinimide

The reaction in the step described above can be performed in the presence or absence of BF₃ and/or N-halosuccinimide. However, it is preferred to perform the reaction in the absence of BF₃ and N-halosuccinimide.

Each component may be added to the reaction system in the above step all at once, in several batches, or continuously.

### Solvent

The reaction of the above step may be performed in the presence or absence of a solvent.

The solvent may be a nonpolar solvent or a polar solvent.

Examples of the solvent include esters, ketones, aromatic hydrocarbons, alcohols, ethers, amines, nitrogen-containing polar organic compounds, nitriles, halogenated hydrocarbons, aliphatic hydrocarbons, fluorine-based solvents, carbonates, other solvents, and combinations thereof.

Examples of esters as the solvent include ethyl acetate, butyl acetate, amyl acetate, ethylene glycol monomethyl ether acetate, and propylene glycol monomethyl ether acetate, and preferably ethyl acetate.

Examples of ketones as the solvent include acetone, methyl ethyl ketone, diethyl ketone, hexanone, methyl isobutyl ketone, heptanone, diisobutyl ketone, acetonylacetone, methylhexanone, acetophenone, cyclohexanone, and diacetone alcohol, and preferably acetone.

Examples of aromatic hydrocarbons as the solvent include benzene, toluene, xylene, and ethylbenzene, and preferably benzene and toluene.

Examples of alcohols as the solvent include methanol, ethanol, n-propanol, isopropanol, n-butanol, pentanol, hexanol, ethylene glycol, diethylene glycol, triethylene glycol, tetraethylene glycol, polyethylene glycol, propylene glycol, dipropylene glycol, tripropylene glycol, polypropylene glycol, trimethylene glycol, and hexanetriol, and preferably methanol and ethanol.

Examples of ethers as the solvent include diethyl ether, dibutyl ether, tetrahydrofuran, tetrahydropyran, dioxane, dimethoxyethane, diethylene glycol diethyl ether, ethylene glycol monomethyl ether, ethylene glycol monoethyl ether, diethylene glycol monomethyl ether, diethylene glycol monoethyl ether, propylene glycol monomethyl ether (PGME; also known as "1-methoxy-2-propanol"), propylene glycol monoethyl ether, triethylene glycol dimethyl ether, triethylene glycol diethyl ether, tetraethylene glycol dimethyl ether, tetraethylene glycol diethyl ether, and anisole, and preferably diethyl ether and tetrahydrofuran.

Examples of amines as the solvent include monoethanolamine, diethanolamine, and triethanolamine.

Examples of nitrogen-containing polar organic compounds as the solvent include N,N-dimethylformamide, N,N-dimethylacetamide, N-methyl-2-pyrrolidone, 2-pyrrolidone, and 1,3-dimethyl-2-imidazolidinone, and preferably N,N-dimethylformamide, N,N-dimethylacetamide, and N-methyl-2-pyrrolidone.

Examples of nitriles as the solvent include acetonitrile, propionitrile, butyronitrile, isobutyronitrile, benzonitrile, and adiponitrile, and preferably acetonitrile.

Examples of halogenated hydrocarbons as the solvent include dichloromethane, dichloroethane, chloroform, carbon tetrachloride, tetrachloroethane, trichloroethane, chlorobenzene, dichlorobenzene, and chlorotoluene, and preferably dichloromethane and chloroform.

Examples of aliphatic hydrocarbons as the solvent include hexane, cyclohexane, heptane, octane, nonane, decane, undecane, dodecane, and mineral spirits, and preferably cyclohexane and heptane.

Examples of fluorine-based solvents include perfluorobenzene, trifluorotoluene, ditrifluorobenzene, and trifluoroethanol, and preferably perfluorobenzene and trifluoroethanol.

Examples of carbonates as the solvent include tetralin dimethyl carbonate, methyl ethyl carbonate, diethyl carbonate, ethylene carbonate, and propylene carbonate, and preferably ethylene carbonate and propylene carbonate.

Examples of other solvents include acetic acid, pyridine, dimethylsulfoxide, sulfolane, and water.

The solvent may be preferably at least one member selected from the group consisting of aliphatic hydrocarbons, aromatic hydrocarbons, halogenated hydrocarbons, ethers, esters, nitriles, and water, more preferably at least one member selected from the group consisting of aliphatic hydrocarbons, halogenated hydrocarbons, ethers, and water, and even more preferably a halogenated hydrocarbon.

These solvents may be used singly, or in a combination of two or more.

The amount of solvent used may be, for example, generally within the range of 0 to 200 parts by mass, preferably within the range of 0 to 100 parts by mass, and more preferably within the range of 0 to 50 parts by mass, per part by mass of compound (1).

### Temperature and Time

The temperature of the above step may be generally within the range of -78 to 200°C, preferably within the range of -10 to 100°C, more preferably within the range of 0 to 100°C, and even more preferably within the range of 10 to 40°C.

The time of the above step may be generally within the range of 0.1 to 72 hours, preferably within the range of 0.5 to 48 hours, and more preferably within the range of 1 to 36 hours.

The production method according to the present disclosure may further comprise isolating or purifying compound (1). Isolation or purification of compound (1) can be performed by, for example, filtration, extraction, dissolution, concentration, precipitation, dehydration, adsorption, or chromatography, or a combination of these methods.

### Halogenating Agent

The halogenating agent according to the present disclosure contains fluorine source (A) and halogen source (B) other than fluorine. Fluorine source (A) and halogen source (B) are as described above in section "Method for Producing Halogen Adduct of Compound (1)."

The molar ratio of fluorine source (A) to halogen source (B) is not limited. The lower limit of the fluorine source (A) content can be, for example, 1 mole or more, preferably 1.5 moles or more, and even more preferably 2 moles or more, per mole of halogen source (B). The upper limit of the fluorine source (A) content can be, for example, 1000 moles or less, preferably 500 moles or less, and even more preferably 300 moles or less, per mole of halogen source (B). The fluorine source (A) content can be, for example, within the range of 1 to 1000 moles, preferably within the range of 1.5 to 500 moles, and more preferably within the range of 2 to 300 moles, per mole of halogen source (B).

The halogenating agent according to the present disclosure may contain BF₃ and/or N-halosuccinimide, but preferably does not contain BF₃ and N-halosuccinimide.

The halogenating agent according to the present disclosure can be suitably used to add fluorine and a halogen other than fluorine to the double bond or triple bond of compound (1) .

### Examples

One embodiment of the present disclosure is described in more detail below with reference to Examples; however, the present disclosure is not limited thereto.

A solution of the substrate listed in Table 1 (1 mmol) in dichloromethane (6 mL) was cooled to 0°C in an ice bath, and HF•pyridine (0.2 mL, 10 mmol HF) was added. Subsequently, NaOCl•5H₂O (298 mg, 4 mmol) was added at once with vigorous stirring. The reaction solution was stirred at 0°C for 2 hours. An aqueous solution of sodium thiosulfate (1.3 g) and water (20 mL) was added to the reaction solution, and the mixture was neutralized with NaHCO₃ and then extracted with dichloromethane (20 mL x 3). The extracted dichloromethane solution was dried over anhydrous sodium sulfate, and the solvent was distilled off under reduced pressure. The yield of the main product was determined by ¹⁹F NMR. The main product was separated by silica gel column chromatography.

**Table 1**

| Substrate | Main product | Yield (%) |
|---|---|---|
| 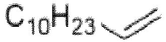 | 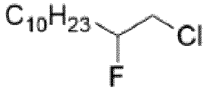 | 81 |
| 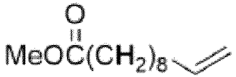 | 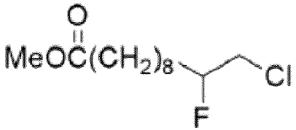 | 78 |
| 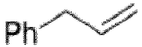 | 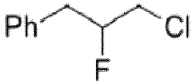 | 64 |
| 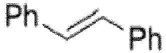 | 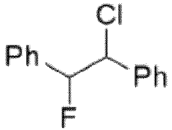 | 70 |
| 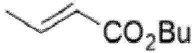 | 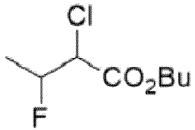 | 46 (0°C) 67 (room temperature) |
| 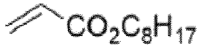 | 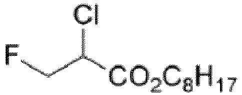 | 47 (room temperature, DCM/HFIP) |
| 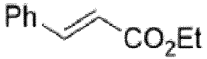 | 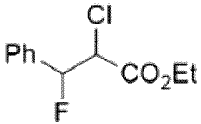 | 85 |

A solution of the substrate listed in Table 2 (1 mmol) in dichloromethane (6 mL) was cooled to 0°C in an ice bath, and HF•pyridine (0.2 mL, 10 mmol HF) was added. Subsequently, tert-BuOCl (0.45 mL, 4 mmol) was added thereto, and the reaction solution was stirred at 0°C for 2 hours. An aqueous solution of sodium thiosulfate (1.3 g) and water (20 mL) was added to the reaction solution, and the mixture was neutralized with NaHCO₃ and then extracted with dichloromethane (20 mL x 3). The extracted dichloromethane solution was dried over anhydrous sodium sulfate, and the solvent was distilled off under reduced pressure. The yield of the product was determined by ¹⁹F NMR. The main product was separated by silica gel column chromatography.

**Table 2**

| Substrate | Main product | Yield (%) |
|---|---|---|
| 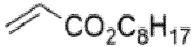 | 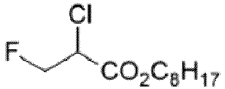 | 83 |
| 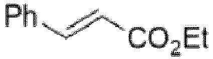 | 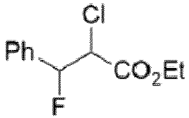 | 100 |
| 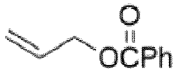 | 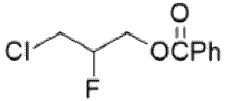 | 72 |
| 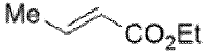 | 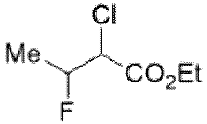 | 98 (room temperature) |

The Examples above demonstrate examples with the use of compound (1) with a double bond as a substrate. However, the present invention is not limited to these. Compound (1) with a triple bond basically tends strongly to have a small steric hindrance and tends to be more reactive than compound (1) with a double bond. Therefore, the results of the Examples above suggest that the reaction will proceed in a similar manner even with the use of compound (1) with a triple bond as a substrate.

## Claims

1. A method for producing a halogen adduct of a compound represented by formula (1): 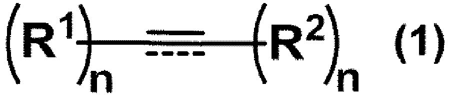 wherein
R¹ and R² are each independently a hydrogen atom, a halogen atom, or an organic group, or R¹ and R² optionally form a ring together with the two adjacent carbon atoms;
n is 1 or 2; and
the symbol 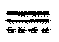 is a double bond or a triple bond, with the proviso that
when the symbol is a triple bond, n is 1,
when the symbol is a double bond, n is 2,
two R¹s are optionally the same or different,
two R²s are optionally the same or different, or
two R¹s or two R²s optionally form a ring together with their adjacent carbon atom,
the method comprising reacting the compound represented by formula (1) with
(A) at least one fluorine source selected from the group consisting of hydrogen fluoride, hydrogen fluoride salts, and fluoride salts, and
(B) a halogen source other than fluorine represented by the formula: R³(OX)ₘ, wherein R³ is a hydrogen atom, a cation, or an organic group, X is a halogen atom other than a fluorine atom, and m is an integer corresponding to the valence of R³, to add fluorine and a halogen other than fluorine to the double bond or triple bond.

2. The production method according to claim 1, wherein the fluorine source (A) is at least one member selected from the group consisting of hydrogen fluoride, hydrogen fluoride amine salts, and alkali metal fluoride salts.

3. The production method according to claim 1 or 2,
wherein the amount of the fluorine source (A) is within the range of 0.1 to 1000 moles, per mole of the compound represented by formula (1).

4. The production method according to any one of claims 1 to 3, wherein in the halogen source (B) other than fluorine, R³ is an alkali metal, an alkaline earth metal, an alkyl group optionally having one or more substituents, a cycloalkyl group optionally having one or more substituents, an aryl group optionally having one or more substituents, a non-aromatic heterocyclic group optionally having one or more substituents, or a heteroaryl group optionally having one or more substituents.

5. The production method according to any one of claims 1 to 4, wherein in the halogen source (B) other than fluorine, X is a chlorine atom.

6. The production method according to any one of claims 1 to 5, wherein the halogen source (B) other than fluorine is represented by the formula: R^{3a}ClO, wherein R^{3a} is an alkali metal or a C₁-C₄ alkyl group.

7. The production method according to any one of claims 1 to 6, wherein the amount of the halogen source (B) other than fluorine is within the range of 0.1 to 10 moles, per mole of the compound represented by formula (1).

8. The production method according to any one of claims 1 to 7, wherein R¹ and R² are each independently a hydrogen atom, a halogen atom, an alkyl group optionally having one or more substituents, an alkoxy group optionally having one or more substituents, an alkylcarbonyl group optionally having one or more substituents, an alkylcarbonyloxy group optionally having one or more substituents, an alkoxycarbonyl group optionally having one or more substituents, a cycloalkyl group optionally having one or more substituents, an aryl group optionally having one or more substituents, or an aralkyl group optionally having one or more substituents, or R¹ and R² optionally form a ring together with the two adjacent carbon atoms.

9. The production method according to claim 8, wherein the one or more substituents are at least one member selected from the group consisting of halo groups, a hydroxyl group, alkoxy groups, alkylcarbonyl groups, alkylcarbonyloxy groups, alkoxycarbonyl groups, arylcarbonyloxy groups, aryloxycarbonyl groups, and aryl groups.

10. The production method according to any one of claims 1 to 9, wherein the compound represented by formula (1) is any one of compounds represented by formulas (1a) to (1c): 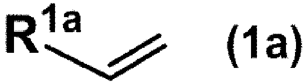 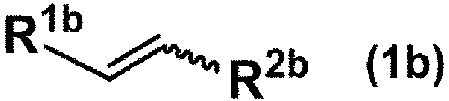 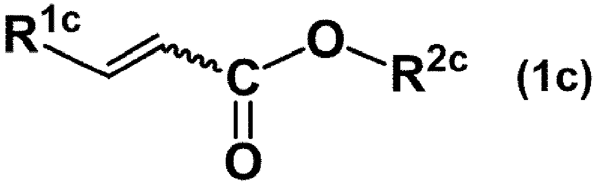 wherein
R^{1a} is an alkyl group optionally having one or more substituents, an aryl group optionally having one or more substituents, or an aralkyl group optionally having one or more substituents,
R^{1b} and R^{2b} are each independently an alkyl group optionally having one or more substituents, an aryl group optionally having one or more substituents, or an aralkyl group optionally having one or more substituents,
R^{1c} is a hydrogen atom, an alkyl group optionally having one or more substituents, an aryl group optionally having one or more substituents, or an aralkyl group optionally having one or more substituents, and
R^{2c} is an alkyl group optionally having one or more substituents.

11. The production method according to any one of claims 1 to 10, wherein the reaction is performed in the presence of a solvent.

12. The production method according to claim 11, wherein the solvent is at least one member selected from the group consisting of aliphatic hydrocarbons, aromatic hydrocarbons, halogenated hydrocarbons, ethers, esters, nitriles, and water.

13. A halogenating agent comprising (A) and (B) below:
(A) at least one fluorine source selected from the group consisting of hydrogen fluoride, hydrogen fluoride salts, and fluoride salts; and
(B) a halogen source other than fluorine represented by the formula: R³(OX)ₘ, wherein R³ is a cation or an organic group, X is a halogen atom other than a fluorine atom, and m is an integer corresponding to the valence of R³.
